# FASCICULE DE BREVET EUROPEEN

(11) **EP 2 079 473 B1**
(45) Date de publication et mention de la délivrance du brevet: **02.04.2014**
(21) Numéro de dépôt: 07858566.8
(22) Date de dépôt: 12.10.2007
(51) Int. Cl.: A61K 31/7048, A61K 9/107, A61P 17/10, A61P 17/08

(54) **Composition dermatologique comprenant des nanocapsules d' avermectine, son procédé de préparation et son utilisation**
Dermatologische Zusammensetzungen mit Avermectin-Nanokapseln, Verfahren zu Ihrer Herstellung und ihre Verwendung
Dermatological composition containing avermectin nanocapsules, method for preparing the same and uses thereof

(30) Priorité: 12.10.2006 FR 0654237
(43) Date de publication de la demande: 22.07.2009
(60) Demande divisionnaire: 11174630.1
(73) Titulaire: Galderma S.A., 6330 Cham (CH)
(72) Inventeur: BAUDONNET, Lara, 31470 Fontenilles (FR); MALLARD, Claire, F-06250 Mougins (FR)
(74) Mandataire: Casalonga
(86) Numéro de dépôt international: PCT/FR2007/052138
(87) Numéro de publication internationale: WO 2008/043973

(56) Documents cités:
- EP-A1- 1 016 453
- WO-A-00/74489
- WO-A-2004/093886
- WO-A-2005/027872
- FR-A1- 2 805 761
- US-A1- 2005 048 088
- US-A1- 2005 079 131
- US-A1- 2005 208 083
- DATABASE WPI Week 200377 Derwent Publications Ltd., London, GB; AN 2003-826098 XP002433269 & KR 2003 037 828 A (LG HOUSEHOLD & HEALTH CARE LTD) 16 mai 2003 (2003-05-16)
- DATABASE WPI Week 200455 Derwent Publications Ltd., London, GB; AN 2004-562833 XP002433270 & CN 1 491 551 A (UNIV HEBEI SCI & TECHNOLOGY) 28 avril 2004 (2004-04-28) cité dans la demande

## Description

La présente invention se rapporte à une composition pharmaceutique, notamment dermatologique, à base d'un composé de la famille des avermectines sous forme d'une nanoémulsion comprenant des nanocapsules huileuses dispersées dans une phase aqueuse. Elle porte également sur son procédé de préparation et sur son utilisation dans la préparation d'un médicament destiné au traitement des affections dermatologiques, en particulier de la rosacée.

L'ivermectine est un mélange de deux composés appartenant à la classe des avermectines, la 5-0-demethyl-22,23-dihydroavermectine A₁ₐ et la 5-0-demethyl-22,23-dihydroavermectine A_{1b}. Ils sont également connus sous le nom de 22,23-dihydroavermectine B₁ₐ et 22,23-dihydroavermectine B_{1b}. L'ivermectine contient au moins 80% de 22,23-dihydroavermectine B₁ₐ et moins de 20% de 22,23-dihydroavermectine B_{1b}. Cet agent actif fait partie de la classe des avermectines, un groupe de lactones macrocycliques produit par le bacterium *Streptomyces avermitilis* (Reynolds JEF (Ed) (1993) Martindale. The extra pharmacopoeia. 29th Edition. Pharmaceutical Press, London). Les avermectines incluent notamment l'ivermectine, l'invermectine, l'avermectine, l'abamectine, la doramectine, l'eprinomectine et la sélamectine.

L'ivermectine est plus particulièrement un antihelminthique. Il est déjà décrit chez l'homme dans le traitement de l'onchocercose à Onchocerca volvulus, de la strongyloïdose (anguillulose) gastro-intestinale (produit Stromectol®), de la gale sarcoptique humaine (Meinking TL et al., N Engl J Med 1995 Jul 6;333(1):26-30 The treatment of scabies with ivermectin) ainsi que dans le traitement de la microfilarémie diagnostiquée ou suspectée chez les sujets atteints de la filariose lymphatique due à Wuchereria bancrofti.

L'ivermectine présente une grande instabilité à la présence d'eau, et il s'avère particulièrement difficile d'obtenir des compositions pharmaceutiques stables la contenant. En effet, ce principe actif présente une très faible solubilité dans l'eau (0.005mg/ml), et se dégrade en milieu hydrophile. Cette sensibilité aux milieux aqueux peut donc conduire à une instabilité chimique de l'actif et/ou à une cristallisation de l'actif initialement solubilisé, et limite sa formulation dans des compositions cosmétiques ou dermatologiques appliquées par voie topique ou orale.

La demande de brevet WO2004/093886 décrit des compositions pharmaceutiques topiques à base d'ivermectine sous forme d'émulsion de crème de lotion de gel ou de solution pour le traitement de la rosacée.
Le brevet US 4,389,397 montre les travaux mis en oeuvre pour augmenter la solubilité de l'ivermectine dans un milieu aqueux, et propose notamment de solubiliser l'ivermectine dans un mélange de tensioactif et de co-solvants organiques.
Par ailleurs, d'autres concepts ont été avancés, comme la solubilisation du principe actif dans une phase grasse (voir la demande FR0603452) pour améliorer la stabilité de cet actif.

Cependant, afin de s'affranchir d'une dégradation de l'ivermectine dans une formulation aqueuse pour application cutanée, il semble intéressant de travailler sur la structure de l'interface entre le milieu de solubilisation de l'ivermectine et la phase aqueuse.

Le brevet CN 1491551 présente des formulations de type nanocapsules d'ivermectine en suspension dans l'eau réalisées via des émulsions polymériques, i.e. un procédé sans solvant mettant en oeuvre une polymérisation in-situ de monomère.

De même, le brevet FR 2 805 761 présente des nanocapsules lipidiques contenant des phosphatidylcholines en association avec un co-tensioactif hydrophile dérivé de polyéthylèneglycol, le Solutol HS 15. Cependant, la présence de co-tensioactif est nécessaire à la réalisation des nanocapsules. Par ailleurs, le procédé de préparation de telles nanocapsules se fait par inversion de phase (procédé TIP), ce qui engendre l'utilisation de cycles de température dans le procédé. Enfin, les actifs sont solubilisés dans une huile composée de triglycérides à chaîne moyenne des acides capryliques et capriques, commercialisée sous le nom de Labrafac WL1 349 par GATTEFOSSE.

Le brevet US 2005/079131 présente quant à lui des émulsions qui contiennent des nanoparticules comprenant des mélanges surfactant/lipide qui comprennent notamment du cholestérol et des dérivés de phosphatidyléthanolamine, ces compositions étant utiles pour l'imagerie et la thérapie dans le but d'améliorer la qualité des images.
La demande de brevet WO00/74489 décrit des compositions stables biocides qui peuvent se présenter sous forme d'émulsions classiques comprenant un principe actif et au moins deux tensio-actifs distincts des lécithines.

La demande de brevet EP 1 016 453 décrit des nanoémulsions comprenant au moins un tensio-actif choisi parmi les éthers gras éthoxylés et les esters gras éthoxylés.

Or la Demanderesse a, de façon surprenante, découvert que des compositions sous forme de nanoémulsions comprenant de l'ivermectine solubilisée dans la phase huileuse dispersée permettaient de stabiliser chimiquement cet actif, et sont faciles à préparer. De telles nanoémulsions comprennent l'ivermectine sous forme solubilisée dans un environnement hydrophile, ne nécessitent pas l'utilisation de polymère ou de solvant organique, et garantissent la stabilité chimique de l'actif. Elles peuvent également favoriser la pénétration cutanée de l'ivermectine, ce qui est utile dans le traitement d'affections dermatologiques, notamment la rosacée.

La présente invention a donc pour objet une composition, notamment pharmaceutique, comprenant au moins un composé de la famille des avermectines, ladite composition étant sous forme de nanoémulsion.
Les composés de la famille des avermectines sont notamment choisis parmi l'ivermectine, l'invermectine, l'avermectine, l'abamectine, la doramectine, l'eprinomectine et la sélamectine, et de préférence l'ivermectine.
La présente invention a donc pour objet une composition présentant une stabilité physique ainsi qu'une stabilité chimique de l'actif dans le temps.

Par stabilité physique selon l'invention, on entend une composition ne présentant aucune modification d'aspect macroscopique (séparation de phase, changement de couleur d'aspect, etc..) ni microscopique (recristallisation des actifs) après stockage aux températures de 4°C et 40°C, pendant 2, 4, 8, 12 semaines.

Par stabilité chimique selon l'invention, on entend une composition dans laquelle la teneur en principe actif reste stable après trois mois à température ambiante et à 40 °C. Une teneur stable en principe actif signifie selon l'invention que la teneur présente très peu de variation par rapport à la teneur initiale, c'est-à-dire que la variation de teneur en principe actif au temps T ne doit pas être inférieure à 90% et plus particulièrement à 95% de la teneur initiale à T0.
La composition selon l'invention comprend de 0,01 à 10 % d'au moins une avermectine en poids par rapport au poids total de la composition. De manière préférentielle, la composition selon l'invention contient de 0.1 à 5% d'au moins une avermectine, de préférence l'ivermectine.

Par nanoémulsion, on entend un système lipidique colloïdal comprenant des nanocapsules lipidiques à interface solide ou semi-solide dispersées dans une phase hydrophile continue, lesdites nanocapsules contenant une phase interne huileuse dans laquelle l'avermectine est solubilisée, et une enveloppe formant l'interface semi-solide ou solide entre la phase interne huileuse et la phase hydrophile continue.

En particulier, la présente invention se rapporte à des nanoémulsions réalisées sans solvant organique.

Selon la présente invention, la composition comprend des nanocapsules et non des nanosphères. On entend par nanocapsules des particules constituées d'un coeur (phase interne) liquide ou semi-liquide à température ambiante, enrobé d'un film (enveloppe ou couche) solide à température ambiante, par opposition à des nanosphères qui sont des particules matricielles, i.e. dont la totalité de la masse est solide. Lorsque les nanosphères contiennent un principe actif pharmaceutiquement acceptable, celui-ci est finement dispersé dans la matrice solide.
On entend par nanocapsule lipidique une nanocapsule dont le coeur est composé d'un ou plusieurs corps gras liquide(s) ou semi-liquide(s) à température ambiante, et dont le film (enveloppe) est de nature lipophile et non polymérique. En effet, les nanocapsules lipidiques ne nécessitent aucun polymère et donc pas de polymérisation in situ.

Par température ambiante, on entend une température comprise entre 15 et 25°C.

Les nanocapsules lipidiques selon la présente invention ont une taille moyenne inférieure à 200nm, de préférence inférieure à 150nm.
Les nanocapsules lipidiques (appelées simplement 'nanocapsules' par la suite) sont présentes dans la composition selon l'invention en quantité comprise entre 10 et 30% en poids par rapport au poids total de la composition, de préférence entre 10 et 20%.

Les nanocapsules sont chacune constituées d'un coeur liquide ou semi-liquide à température ambiante, enrobé d'un film solide à température ambiante.

Le film (couche) enrobant les nanocapsules est le seul constituant solide à température ambiante de la formulation. Il n'est pas de nature polymérique. Il est constitué par un ou plusieurs surfactants lipophiles choisis parmi la famille des lécithines; le surfactant lipophile est une lécithine hydrogénée dont le pourcentage de phosphatidylcholine saturée (ou hydrogénée) est élevé. Par pourcentage élevé, on entend une quantité de 70 à 99% de phosphatidylcholine saturée (ou hydrogénée) par rapport au poids total de lécithine. Les phosphatidylcholines montrent de bonnes compatibilités avec la peau avec un très faible potentiel irritant.
La lécithine utilisée dans la présente invention étant solide à température ambiante, cela favorise la formation d'une interface semi-solide dans la nanoémulsion.

Comme lécithines utilisables, on peut citer notamment les lécithines de soja ou d'oeuf, naturelles ou synthétiques, ayant une teneur en phosphatidylcholine hydrogénée supérieure à 70%, comme par exemple le LIPOID de grade S75-3, S100-3 ou SPC-3, l'Epikuron de grade 200 SH ou 100H, ou le Phospholipon de grade 80H, 90H ou 100H.
Le film de surfactant lipophile enrobant les nanocapsules tel que défini ci-dessus est présent en quantité comprise entre 0.1 et 10% en poids, de préférence entre 1 et 5% en poids par rapport au poids total de la composition.

Le film de surfactant lipophile, notamment de lécithine, selon l'invention permet à lui seul l'encapsulation de l'avermectine, de préférence l'ivermectine, ce qui évite te contact de cette avermectine avec la phase hydrophile, et ainsi assure la stabilité chimique de cet actif. En particulier, la composition, et notamment le film, ne contient aucun co-tensioactif distinct des lécithines, et notamment aucun co-tensioactif hydrophile.

L'avermectine se trouve ainsi solubilisée dans le coeur des nanocapsules (phase interne), ledit coeur étant liquide ou semi-liquide à température ambiante.
Plusieurs travaux de préformulation (« Preformulation stability screening of ivermectine with non-ionic emulsion excipient » N.O Shaw, M.M. de Villiers et A.P. Lötter dans Pharmazie 54 (199) pp372-376) ont montré que l'ivermectine est incompatible avec certains excipients lipophiles. Les résultats analytiques présentés dans cette publication montrent une dégradation de l'ivermectine notamment dans le ceteareth-25, le ceteareth-6, le PEG-8-distearate, le PEG-8-stearate, le PEG-660-OH-stearate, le PEG-4000, le polyoxyethylene-10, le glycerol monostearate S/E, le propylene glycol dicaprylate, le cetearyl octanoate, le poly-glycerol-3-diolate, un mélange de C18-C36 triglycérides, la Gamma cyclodextrine, la lécithine de soja, le cholestérol et l'acide stéarique.
En particulier, dans cette publication, la lécithine de soja est citée comme un des excipients pouvant dégrader l'Ivermectine. Or dans la présente invention, les lécithines mises en oeuvre sont préférentiellement des lécithines hydrogénées. Comme le montrent les études réalisées à l'exemple 1, de façon surprenante, de telles lécithines hydrogénées ne dégradent pas l'Ivermectine.

La composition de la phase interne (coeur liquide ou semi-liquide à température ambiante) est donc essentielle pour la stabilité du principe actif. La demanderesse a montré, notamment dans l'exemple 1, que lorsque le coeur est essentiellement constitué d'un corps gras particulier liquide ou semi-liquide à température ambiante dans lequel l'avermectine est solubilisée, la stabilité de l'actif est maintenue. Un tel corps gras est notamment le diisopropyl adipate, commercialisé sous le nom Crodamol DA par Croda, ou sous le nom de Ceraphyl 230 par ISP, ou sous le nom de Wickenol 116 par Alzo, le PPG 15 stearyl ether vendu sous le nom d'Arlamol E par la société Uniqema, l'octyl dodecanol vendu sous le nom d'Eutanol G par la société Cognis, le benzoate d'alkyle C12-C15 vendu sous le nom de Tegosoft TN par la société Degussa, le dicaprylyl éther vendu sous le nom de Cetiol OE, l'octyl palmitate vendu sous le nom Crodamol OP, l'éthoxydiglycol vendu sous le nom de Transcutol HP, la lanoline, le benzoate de benzyle et leurs mélanges.
En sus de ce(s) corps gras, la phase interne peut également comprendre un ou plusieurs corps gras liquides ou semi-liquides à température ambiante non solubilisants de l'actif, tels que notamment les triglycérides ayant de 18 à 36 atomes de carbone, l'alcool cétylique vendu sous le nom de Speziol C16 par la société Cognis.
Préférentiellement le corps gras de la phase interne est uniquement constitué de diisopropyl adipate.

Un tel corps gras est présent en quantité comprise entre 90 et 99.99% en poids par rapport au poids total de la phase interne.

La phase hydrophile continue comprend de l'eau. Cette eau peut être de l'eau déminéralisée, une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène ou l'eau d'Aix les Bains. L'eau peut être présente à une teneur comprise entre 70 et 90 % en poids par rapport au poids total de la composition, de préférence comprise entre 80 et 90 % en poids.

La phase hydrophile peut également comprendre d'autres composés hydrophiles tels que des agents conservateurs ou des humectants.
Parmi les agents conservateurs utilisables, on peut citer notamment les parabens ou le phénoxyéthanol.
Parmi les humectants utilisables, on peut citer notamment la glycérine.

Dans un des modes préférés selon l'invention, la composition peut également comprendre un agent gélifiant. Cet agent gélifiant est de préférence un dérivé cellulosique choisi parmi les gélifiants cellulosiques semi-synthétiques, tels que la méthylcellulose, l'éthylcellulose, l'hydroxypropylméthylcellulose, l'hydroxyéthylcellulose, la carboxyméthylcellulose, l'hydroxyméthylcellulose, et l'hydroxypropylcellulose, pris seuls ou en mélange. On utilise de préférence l'hydroxypropylméthylcellulose ou l'hydroxyéthylcellulose. Ces composés sont notamment commercialisés par la société Dow Chemical sous le nom de Methocel® (par exemple : Methocel® E4M) ou par la société Hercules sous le nom de Natrosol® (par exemple : Natrosol® 250 HHX). L'agent gélifiant peut également être choisi parmi les gommes naturelles comme la gomme de tragacanthe, la gomme de guar, la gomme d'acacia, la gomme arabique, l'amidon et ses dérivés, les copolymères d'acide polyacrylique et de methylmethacrylate, les carboxyvinyl polymères, les polyvinyl pyrrolidones et leurs dérivés, les polyvinyl alcools, l'alginate de sodium, la pectine, la dextrine, le chitosan, pris seuls ou en mélange, les polyacrylamides tel que le mélange polyacrylamide / isoparaffine C13-14 / laureth-7 comme par exemple celui vendu par la société SEPPIC sous le nom de Sepigel 305 ou le mélange acrylamide, AMPS copolymer dispersion 40% / isohexadecane sous le nom de Simulgel 600PHA, ou la famille des amidons modifiés tel que Structure Solanace revendu par National Starch ou leurs mélanges.
L'agent gélifiant est utilisé notamment à une concentration comprise entre 0.1 et 10% en poids, de préférence entre 0.1 et 2% en poids.
La présence d'un gélifiant dans la composition selon l'invention permet d'améliorer la stabilité physique de la composition dans le temps.

La composition pharmaceutique utilisable selon l'invention est destinée au traitement de la peau et peut être administrée par voie topique, parentérale ou orale. De préférence, la composition est administrée par voie topique.

Par voie orale, la composition pharmaceutique peut se présenter sous forme liquide, ou pâteuse, et plus particulièrement sous formes de gélules, de dragées, ou de sirops.

Par voie parentérale, la composition peut se présenter sous forme de suspensions pour perfusion ou pour injection.

Par voie topique, la composition peut se présenter sous forme liquide, ou pâteuse, et plus particulièrement sous forme de crèmes, de laits, de pommades, de tampons imbibés, de syndets, de lingettes, de gels, de sprays, de mousses, de lotions, de sticks, de shampoings, ou de bases lavantes.

La composition sous forme de nanoémulsion comprend ainsi de préférence dans l'eau, en poids par rapport au poids total de la composition :
a) 0.1 à 5% de surfactant lipophile solide à température ambiante, de préférence la lécithine ;
b) 1 à 20% de corps gras liquide ou semi-liquide à température ambiante, de préférence du diisopropyl adipate ;
c) 0.1 à 5% d'au moins une avermectine, de préférence l'ivermectine ;
d) 0 à 2% de gélifiant, de préférence un dérivé cellulosique.

La composition pharmaceutique selon l'invention pourra en outre contenir des additifs inertes ou des combinaisons de ces additifs, tels que
- des agents conservateurs;
- des agents propénétrants ;
- des agents stabilisants ;
- des agents régulateurs d'humidité ;
- des agents régulateurs de pH ;
- des agents modificateurs de pression osmotique ;
- des filtres UV-A et UV-B ;
- et des antioxydants.

Parmi les propénétrants utilisables selon l'invention, on peut notamment citer le propylène glycol, la N-méthyl-2-pyrrolidone, ou encore le diméthylsulfoxyde.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

Ces additifs peuvent être présents dans la composition de 0.001 à 20% en poids par rapport au poids total de la composition.

L'invention a également pour objet un procédé de préparation des nanoémulsions comprenant au moins une avermectine, de préférence l'ivermectine. Ce procédé fait appel à un Homogénéisateur Haute Pression (HHP). En particulier, le procédé selon l'invention n'utilise pas de Température d'Inversion de Phase (TIP) (utilisé notamment dans les brevets FR 2 805 761 et FR 2 840 531), et ne nécessite donc pas de cycle(s) de montée et de descente en température. En effet, le procédé selon l'invention s'effectue dans le HHP à froid ; le HHP ne nécessite donc pas de chauffage et de refroidissement successifs, et n'est pas thermo-régulé.

Le procédé mis en oeuvre dans la présente invention comprend les étapes suivantes :
(i) Solubilisation de l'avermectine dans le corps gras liquide ou semi-liquide à température ambiante, afin d'obtenir la phase huileuse.
   De préférence, l'ivermectine est solubilisée dans le corps gras, par exemple dans le Crodamol DA.
(ii) Mélange des composés hydrophiles, afin d'obtenir la phase hydrophile.
   Notamment, le(s) conservateur(s) est(sont) mélangé(s) à l'eau.
   Les deux phases (lipophile et hydrophile) sont ensuite de préférence chauffées séparément à une température de préférence d'environ 75°C.
(iii) Dispersion du surfactant lipophile dans la phase huileuse obtenue en (i) ou dans la phase hydrophile obtenue en (ii).
   Le surfactant lipophile, notamment la lécithine, est dispersé dans la phase hydrophile ou dans la phase huileuse interne ; par exemple, le Phospholipon 90H est dispersé dans la phase huileuse, tandis que le Lipoid S75-3 est dispersé dans la phase aqueuse.
(iv) Mélange des phases huileuse et hydrophile.
   Une fois les deux phases en température, celles-ci sont mélangées sous agitation. Une fois cette pré-homogénéisation réalisée, l'émulsion est introduite dans l'Homogénéisateur Haute Pression (HHP).
(v) Introduction du mélange obtenu en (iv) dans l'Homogénéisateur Haute Pression, afin d'obtenir une nanoémulsion.
   L'utilisation d'un Homogénéisateur Haute Pression nécessite de fixer le nombre de passages dans la chambre d'homogénéisation et la pression d'homogénéisation. Le procédé d'Homogénéisation est alors appliqué :
   - minimum 500 bars jusqu'à 1000 bars de pression d'homogénéisation dans la chambre d'homogénéisation,
   - entre 5 et 10 passages dans la chambre d'homogénéisation.

   Au cours des passages dans la chambre d'homogénéisation, la nanoémulsion n'est pas chauffée, le système HHP n'est pas contrôlé en température.
(vi) Gélification : optionnellement, ajout d'un agent gélifiant à la composition obtenue en (v).
   Quand elle a lieu, l'étape de gélification de la nanoémulsion intervient en fin de fabrication après les différents passages dans l'HHP, au cours du refroidissement de la nanoémulsion.
   L'agent gélifiant est alors ajouté sous agitation suffisante à une dispersion homogène, au cours du refroidissement de la nanoémulsion. L'agitation est maintenue le temps nécessaire à achever l'étape de gélification du système.

L'invention a également pour objet l'utilisation de la nanoémulsion selon l'invention pour la préparation d'un médicament destiné à traiter les affections dermatologiques humaines.

L'utilisation de la nanoémulsion selon l'invention comme médicament, est particulièrement destinée au traitement de la rosacée, de l'acné vulgaire, de la dermite seborrhéique, de la dermatite periorale, des éruptions acneiformes, de la dermatite acantholytique transitoire, et de l'acné miliaris necrotica.

L'utilisation de la nanoémulsion selon l'invention est plus particulièrement destinée au traitement de la rosacée.

Il va être maintenant donné, à titre d'illustration et sans aucun caractère limitatif, diverses formulations de compositions comprenant des avermectines naturelles.

### Exemple 1 : Etude de préforrnulation de l'ivermectine

Il existe de nombreux excipients utilisés dans la formulation d'émulsions ou de liposomes qui génèrent des dégradations de l'ivermectine. Le tableau ci-dessous présente les résultats de tests de stabilité et de solubilité de l'ivermectine dans divers excipients :

| | Solubilité max (mg/g) | T0 (mg/g) | T1 mois | | T3 mois | |
|---|---|---|---|---|---|---|
| | | | Tambiante | 40 °C | Tambiante | 40 °C |
| Solutol HS15 (PEG-15 hydroxystearate) | 10.8 | 9.8 | 9.4 (95%) | 7.1 (72%) | NR | NR |
| Alcool Benzylique | 32.8 | 9.9 | 9.8 (99%) | 9.7 (98%) | NR | NR |
| Labrasol (PEG-8 glycérides capryliques/capriques) | NR | 8.1 | 8.1 (100%) | 7.9 (98%) | 8.1 (100%) | 5.6 (69%) |
| Labrafac Hydro WL1219 (PEG-4 esters de triglycérides capryliques/ capriques) | 70 | 9.0 | 8.5 (95%) | 6.7 (75%) | 6.3 (70%) | 4.2 (47%) |
| Miglyol 812 | 17.9 | 8.7 | 8.3 (95%) | 8.2 (94%) | 8.3 (96%) | 8.4 (96%) |
| Lipoid S75-3+ Alcool Benzylique | NR | 9.4 | 9.5 (101 %) | 9.5 (101%) | 9.2 (98%) | 9.7 (103%) |
| Ph90H+Alcool Benzylique | NR | 9.25 | 9.7 (105%) | 9.5 (103%) | | |
| Crodamol DA | 94.2 | Stable sur 1 mois | | | | |
| Benzyl Benzoate | 44.2 | NR | | | | |

| | | | | | | |
|---|---|---|---|---|---|---|
| Ph90H : Phospholipon 90H NR = Non réalisé | | | | | | |

D'après le tableau ci-dessus, il n'est pas possible d'utiliser le Solutol HS15 dans une nanoémulsion contenant de l'ivermectine. En effet, le tableau ci-dessus montre une dégradation à 1 mois de 28% de l'ivermectine. Par ailleurs, il apparaît que l'ivermectine d'une part n'est pas parfaitement stable dans le Mygliol 812, qui correspond à une huile composée de triglycérides à chaîne moyenne des acides capryliques et capriques (C8 et C10), et d'autre part présente une solubilité relativement faible dans cette huile.
En revanche, il semble possible de formuler l'ivermectine dans une nanoémulsion avec la lécithine de soja (LIPOID S75-3) et le diisopropyl adipate (Crodamol DA) pour assurer la stabilité chimique de cet actif. Les choix de la phase huileuse interne et du surfactant à utiliser sont donc validés.

### Exemple 2 : Formulations

| Constituants | Formulation 1 | Formulation 2 | Formulation 3 | Formulation 4 |
|---|---|---|---|---|
| Ivermectine | 1.1% | 1.1% | 1% | 1% |
| Crodamol DA | 13.9% | 13.9% | 14% | 14% |
| Phospholipon 90H | | 1.9% | | 1.9% |
| Lipoid S75-3 | 1.9% | | 1.9% | |
| Nipagin N M | 0.2% | 0.2% | 0.2% | 0.2% |
| Agent Gélifiant - par exemple Dérivé cellulosique : Natrosol 250 HHX | | | 0.5% | 0.5% |
| Eau | Qsp 100% | Qsp 100% | Qsp 100% | Qsp 100% |

### Exemple 3 : Procédé de fabrication des formulations de l'exemple 2

Le procédé mis en oeuvre dans cette invention utilise un Homogénéisateur Haute Pression (HHP).

### Etapes de fabrication :

### 1. Solubilisation de l'Ivermectine :

L'Ivermectine est solubilisée dans la phase huileuse, ici dans le Crodamol DA.

### 2. Préparation de la phase hydrophile :

Le conservateur est solubilisé dans l'eau.

### 3. Dispersion de la phosphatidylcholine Hydrogénée :

La phosphatidylcholine hydrogénée mise en oeuvre est dispersée dans la phase hydrophile ou dans la phase huileuse en fonction de la teneur en phosphatidylcholine.

Les deux phases sont chauffées séparément à environ 75°C.

### 4. Mélange des phases :

Une fois les deux phases en température, celles-ci sont mélangées sous agitation (homogénéisation turrax 2 minutes à 8000 rpm).

Une fois cette pré-homogénéisation réalisée, l'émulsion est introduite dans le HHP.

### 5. Homogénéisation Haute Pression :

L'utilisation d'un Homogénéisateur Haute Pression nécessite de fixer le nombre de passages dans la chambre d'homogénéisation et la pression d'homogénéisation.
Le procédé d'Homogénéisation est alors appliqué :
minimum 500 bars jusqu'à 1000 bars de pression d'homogénéisation dans la chambre d'homogénéisation,
entre 5 et 10 passages dans la chambre d'homogénéisation.

Au cours des passages dans la chambre d'homogénéisation, la nanoémulsion n'est pas chauffée, le système HHP n'est pas contrôlé en température.

### 6. Gélitication :

Quand elle a lieu, l'étape de gélification de la nanoémulsion intervient en fin de fabrication après les différents passages dans l'HHP, au cours du refroidissement de la nanoémulsion.
L'agent gélifiant est alors ajouté sous agitation suffisante à une dispersion homogène, au cours du refroidissement de la nanoémulsion.
L'agitation est maintenue le temps nécessaire à achever l'étape la gélification du système.

### Exemple 4 : ETUDES DE STABILITE des formulations de l'exemple 2

### 1- PHYSIQUE

Analyse granulométrique : Zetasizer : Nanoseries-Nano-ZS (Malvern)
Deux dilutions sont utilisées pour effectuer les analyses granulométriques :
1 d : l0ul de la nanoémulsion dans 15 ml d'eau distillée filtrée
2d : 1 mL de 1d dans 5 mL d'eau distillée

### Formulation 1 (Lipoid S75-3)

| | | | | | | |
|---|---|---|---|---|---|---|
| T0 | Taille (nm) | 141 | | | | |
| | CV % | 5% | | | | |
| Stabilité en Température | | 4°C | Tambiante | | 40°C | |
| T 3 mois | Taille (nm) | 133 | 124 | 442 | 131 | 485 |
| | CV % | 6 | 13 | 20 | 17 | 20 |
| | % en nombre | 100% | >99% | <1% | >95% | <5% |

| | | | | | | |
|---|---|---|---|---|---|---|
| CV = Coefficient de Variation | | | | | | |

Il existe deux populations granulométriques qui ne sont pas toujours détectées au cours des mesures en fonction des dilutions analysées. L'apparition de la population entre 400 et 800nm n'est pas reproductible. (CV important pour dilution 1 d et disparition pour dilution 2d)

### Formulation 2 (Phospholipon 90H)

| | | | | | | | |
|---|---|---|---|---|---|---|---|
| T0 | Taille (nm) | 157 | | | | 600 | |
| | CV % | 6.5 | | | | 25 | |
| | % en nombre | >95% | | | | <5% | |
| Stabilité en Température | | 4°C | | Tambiante | | 40 °C | |
| T1 mois | Taille (nm) | 150 | 309 | 133 | 650 | 250 | 433 |
| | CV% | 11 | 20 | 9 | 18 | 9 | 15 |
| | % en nombre | >95% | <5% | >95% | <5% | >95% | <5% |
| T 2mois | Taille (nm) | 242 | 479 | 221 | 630 | 159 | 519 |
| | CV % | 11 | 10 | 8 | 10 | 2 | 10 |
| | % en nombre | >99% | <1% | >99% | <1% | >95% | <5% |

### Formulations 3 et 4 avec Natrosol 250HHX

La stabilité physique des formulations 3 et 4 gélifiées est réalisée sur la base d'observations microscopiques à TO, 6 et 9 mois aux températures de 4°C, ambiante et 40°C.
Même après 9 mois de stabilité aux 3 températures, les formulations 3 et 4 ne présentent aucune dégradation et montrent une taille de gouttelette plus fine que les compositions sans gélifiants.
La stabilité physique des compositions selon l'invention semble donc améliorée avec l'augmentation de la viscosité de la composition par ajout du gélifiant.

### 2- CHIMIQUE

Dosage chimique des compositions selon l'invention.

### Formulation 1 (Lipoid S75-3)

| | | | | |
|---|---|---|---|---|
| T0 | mg/g | 10.92 | | |
| | % Titre attendu | 102 | | |
| | | 4°C | Tambiante | 40°C |
| T 1mois | % Titre attendu | 105 | 104 | 105 |
| T 2mois | % Titre attendu | 104 | 107 | 106 |
| T 3mois | % Titre attendu | | 108 | 102 |

Cette formulation est stable chimiquement sur 3 mois pour les 3 conditions de température.

### Formulation 3 (Lipoid S75-3+ Natrosol)

| | | | | |
|---|---|---|---|---|
| T0 | mg/g | 10.29 | | |
| | % Titre attendu | 96 | | |
| | | 4°C | Tambiante | 40°C |
| T 1mois | % Titre attendu | NR | 103 | 103 |
| T 2mois | % Titre attendu | 105 | 100 | 101 |
| T 3mois | % Titre attendu | 98 | 107 | 81 |

Cette formulation est stable chimiquement sur 3 mois pour les 3 conditions de température.

### Formulation 2 (Phospholipon 90H)

| | | | | |
|---|---|---|---|---|
| T0 | mg/g | 10.93 | | |
| | % Titre attendu | 102 | | |
| | | 4°C | Tambiante | 40 °C |
| T 1mois | % Titre attendu | 99 | 101 | 99 |
| T 2mois | % Titre attendu | 99 | 99 | 107 |
| T 3mois | % Titre attendu | 102 | 101 | 104 |

### Formulation 4 (Phospholipon 90H + Natrosol)

| | | | | |
|---|---|---|---|---|
| T0 | mg/g | 10.59 | | |
| | % Titre attendu | 96 | | |
| | | 4°C | Tambiante | 40°C |
| T 5mois | % Titre attendu | 101 | 96 | 78 |
| T 9mois | % Titre attendu | 100 | 96 | 61 |

Conclusion : L'ivermectine est stable chimiquement sur 3 mois aux 3 conditions de températures : 4°C, température ambiante (Tambiante) et 40°C, dans les nanoémulsions selon l'invention.

## Revendications

1. Composition comprenant au moins un composé de la famille des avermectines, comprenant des nanocapsules lipidiques dispersées dans une phase hydrophile continue, lesdites nanocapsules lipidiques contenant une phase interne huileuse dans laquelle l'avermectine est solubilisée, et un surfactant lipophile solide à température ambiante choisi parmi les lécithines ayant de 70 à 99% en poids de phosphatidylcholine hydrogénée par rapport au poids total de lécithine et qui enrobe ladite phase interne huileuse.

2. Composition selon la revendication 1, **caractérisée en ce qu'**elle ne contient aucun co-tensioactif distinct des lécithines.

3. Composition selon l'une des revendications 1 ou 2, **caractérisée en ce que** le composé de la famille des avermectines est choisi parmi l'ivermectine, l'invermectine, l'avermectine, l'abamectine, la doramectine, l'eprinomectine et la sélamectine.

4. Composition selon l'une des revendications 1 à 3, **caractérisée en ce que** le composé de la famille des avermectines est l'ivermectine.

5. Composition selon l'une des revendications 1 à 4, **caractérisée en ce que** la phase interne huileuse des nanocapsules comprend un corps gras liquide ou semi-liquide à température ambiante.

6. Composition selon l'une des revendications 1 à 5, **caractérisée en ce que** la phase interne huileuse des nanocapsules comprend au moins un corps gras choisi parmi le diisopropyl adipate, le PPG 15 stearyl ether, l'octyl dodecanol, le benzoate d'alkyle C12-C15, le dicaprylyl éther, l'octyl palmitate, l'éthoxydiglycol, la lanoline, le benzoate de benzyle et leurs mélanges.

7. Composition selon la revendication 5 ou 6, **caractérisée en ce que** le corps gras est présent en quantité comprise entre 90 et 99.99% en poids par rapport au poids total de la phase interne.

8. Composition selon l'une des revendications 1 à 7, **caractérisée en ce que** le surfactant lipophile solide à température ambiante est présent en quantité comprise entre 0.1 et 10% en poids par rapport au poids total de la composition, de préférence entre 1 et 5%.

9. Composition selon l'une des revendications 1 à 8, **caractérisée en ce qu'**elle comprend un agent gélifiant.

10. Composition selon la revendication 9, **caractérisée en ce que** l'agent gélifiant est un dérivé cellulosique choisi parmi la méthylcellulose, l'éthylcellulose; l'hydroxypropylméthylcellulose, l'hydroxyéthylcellulose, la carboxyméthylcellulose, l'hydroxyméthylcellulose, et l'hydroxypropylcellulose, pris seuls ou en mélange.

11. Composition selon l'une des revendications 1 à 10, **caractérise en ce qu'**elle comprend dans l'eau, en poids par rapport au poids total de la composition :
a) 0.1 à 5% de surfactant lipophile solide à température ambiante ;
b) 1 à 20% de corps gras liquide ou semi-liquide à température ambiante ;
c) 0.1 à 5% d'au moins une avermectine ;
d) 0 à 2% de gélifiant.

12. Composition selon la revendication 11, **caractérise en ce qu'**elle comprend dans l'eau, en poids par rapport au poids total de la composition :
a) 0.1 à 5% de lécithine ;
b) 1 à 20% de diisopropyl adipate ;
c) 0.1 à 5% d'ivermectine ;
d) 0 à 2% de dérivé cellulosique.

13. Composition selon l'une des revendications 1 à 12, **caractérisée en ce qu'**elle se présente sous une forme adaptée pour une administration topique.

14. Composition selon l'une des revendications 1 à 13, à titre de médicament.

15. Utilisation d'une composition selon l'une des revendications 1 à 13, pour la préparation d'un médicament destiné au traitement d'affections dermatologiques choisies parmi la rosacée, l'acné vulgaire, la dermite seborrhéique, la dermatite periorale, les éruptions acneiformes, la dermatite acantholytique transitoire, et l'acné miliaris necrotica.

16. Utilisation selon la revendication 15, **caractérisée en ce que** l'affection dermatologique est la rosacée.

17. Procédé de préparation d'une composition selon l'une des revendications 1 à 13, **caractérisé en ce qu'**il comprend les étapes suivantes :
(i) Solubilisation de l'avermectine dans un corps gras liquide ou semi-liquide à température ambiante, afin d'obtenir la phase huileuse ;
(ii) Mélange des composés hydrophiles, afin d'obtenir la phase hydrophile ;
(iii) Dispersion du surfactant lipophile dans la phase huileuse obtenue en (i) ou dans la phase hydrophile obtenue en (ii) ;
(iv) Mélange de la phase huileuse avec la phase hydrophile ;
(v) Introduction du mélange obtenu en (iv) dans un Homogénéisateur Haute Pression, afin d'obtenir une nanoémulsion.

18. Procédé selon la revendication 17, **caractérisé en ce qu'**il comprend une étape (vi) d'ajout d'un agent gélifiant à la nanoémulsion obtenue en (v).

## Patentansprüche

1. Zusammensetzung, umfassend mindestens eine Verbindung aus der Familie der Avermectine, umfassend Lipidnanokapseln, die in einer kontinuierlichen hydrophilen Phase dispergiert sind, wobei die Lipidnanokapseln eine ölige innere Phase, in der das Avermectin solubilisiert ist, enthält, und ein bei Umgebungstemperatur festes lipophiles Tensid, das aus Lecithinen mit 70 bis 99 Gew.-% hydriertem Phosphatidylcholin, bezogen auf das Gesamtgewicht von Lecithin, ausgewählt ist und die ölige innere Phase umhüllt.

2. Zusammensetzung nach Anspruch 1, **dadurch gekennzeichnet, dass** es kein von Lecithinen verschiedenes Cotensid enthält.

3. Zusammensetzung nach Anspruch 1 oder 2, **dadurch gekennzeichnet, dass** die Verbindung aus der Familie der Avermectine aus Ivermectin, Invermectin, Avermectin, Abamectin, Doramectin, Eprinomectin und Selamectin ausgewählt ist.

4. Zusammensetzung nach einem der Ansprüche 1 bis 3, **dadurch gekennzeichnet, dass** es sich bei der Verbindung aus der Familie der Avermectine um Ivermectin handelt.

5. Zusammensetzung nach einem der Ansprüche 1 bis 4, **dadurch gekennzeichnet, dass** die ölige innere Nanokapselphase eine bei Umgebungstemperatur flüssige oder halbflüssige Fettsubstanz umfasst.

6. Zusammensetzung nach einem der Ansprüche 1 bis 5, **dadurch gekennzeichnet, dass** die ölige innere Nanokapselphase mindestens eine Fettsubstanz, die aus Diisopropyladipat, PPG-15-Stearylether, Octyldodecanol, Benzoesäure-C12-C15-alkylester, Dicaprylylether, Octylpalmitat, Ethoxydiglykol, Lanolin, Benzoesäurebenzylester und Mischungen davon ausgewählt ist, umfasst.

7. Zusammensetzung nach Anspruch 5 oder 6, **dadurch gekennzeichnet, dass** die Fettsubstanz in einer Menge zwischen 90 und 99,99 Gew.-%, bezogen auf das Gesamtgewicht der inneren Phase, vorliegt.

8. Zusammensetzung nach einem der Ansprüche 1 bis 7, **dadurch gekennzeichnet, dass** das bei Umgebungs-temperatur feste lipophile Tensid in einer Menge zwischen 0,1 und 10 Gew.-%, bezogen auf das Gesamtgewicht der Zusammensetzung, vorzugsweise zwischen 1 und 5%, vorliegt.

9. Zusammensetzung nach einem der Ansprüche 1 bis 8, **dadurch gekennzeichnet, dass** sie ein Geliermittel umfasst.

10. Zusammensetzung nach Anspruch 9, **dadurch gekennzeichnet, dass** es sich bei dem Geliermittel um ein Cellulosederivat handelt, das aus Methylcellulose, Ethylcellulose, Hydroxypropylmethylcellulose, Hydroxyethylcellulose, Carboxymethylcellulose, Hydroxymethylcellulose und Hydroxypropylcellulose alleine oder als Mischung ausgewählt ist.

11. Zusammensetzung nach einem der Ansprüche 1 bis 10, **dadurch gekennzeichnet, dass** sie in Wasser, bezogen auf das Gesamtgewicht der Zusammensetzung:
a) 0,1 bis 5 Gew.-% bei Umgebungstemperatur festes lipophiles Tensid;
b) 1 bis 20 Gew.-% bei Umgebungstemperatur flüssige oder halbflüssige Fettsubstanz;
c) 0,1 bis 5 Gew.-% mindestens eines Avermectins;
d) 0 bis 2 Gew.-% Geliermittel
umfasst.

12. Zusammensetzung nach Anspruch 11, **dadurch gekennzeichnet, dass** sie in Wasser, bezogen auf das Gesamtgewicht der Zusammensetzung:
a) 0,1 bis 5 Gew.-% Lecithin;
b) 1 bis 20 Gew.-% Diisopropyladipat;
c) 0,1 bis 5 Gew.-% Ivermectin;
d) 0 bis 2 Gew.-% Cellulosederivat
umfasst.

13. Zusammensetzung nach einem der Ansprüche 1 bis 12, **dadurch gekennzeichnet, dass** sie in einer für die topische Verabreichung geeigneten Form vorliegt.

14. Zusammensetzung nach einem der Ansprüche 1 bis 13 als Arzneimittel.

15. Verwendung einer Zusammensetzung nach einem der Ansprüche 1 bis 13 zur Herstellung eines Arzneimittels zur Behandlung von dermatologischen Leiden, die aus Rosacea, Acne vulgaris, seborrhoischer Dermatitis, perioraler Dermatitis, akniformen Eruptionen, transitorischer akantholytischer Dermatose und Acne miliaris necrotica ausgewählt sind.

16. Verwendung nach Anspruch 15, **dadurch gekennzeichnet, dass** es sich bei dem dermatologischen Leiden um Rosacea handelt.

17. Verfahren zur Herstellung einer Zusammensetzung nach einem der Ansprüche 1 bis 13, **dadurch gekennzeichnet, dass** es folgende Schritte umfasst:
(i) Solubilisieren des Avermectins in einer bei Umgebungstemperatur flüssigen oder halbflüssigen Fettsubstanz zum Erhalt der öligen Phase;
(ii) Mischen der hydrophilen Verbindungen zum Erhalt der hydrophilen Phase;
(iii) Dispergieren des lipophilen Tensids in der in (i) erhaltenen öligen Phase oder in der in (ii) erhaltenen hydrophilen Phase;
(iv) Mischen der öligen Phase mit der hydrophilen Phase;
(v) Eintragen der in (iv) erhaltenen Mischung in einen Hochdruckhomogenisator zum Erhalt einer Nanoemulsion.

18. Verfahren nach Anspruch 17, **dadurch gekennzeichnet, dass** es einen Schritt (vi) des Zugebens eines Geliermittels zu der in (v) erhaltenen Nanoemulsion umfasst.

## Claims

1. Composition comprising at least one compound of the avermectin family, comprising lipid nanocapsules dispersed in a continuous hydrophilic phase, said lipid nanocapsules containing an oily internal phase in which the avermectin is solubilized, and a lipophilic surfactant which is solid at ambient temperature, chosen from lecithins containing from 70% to 99% by weight of hydrogenated phosphatidylcholine relative to the total weight of lecithin, and which coats said oily internal phase.

2. Composition according to Claim 1, **characterized in that** it contains no cosurfactant distinct from lecithins.

3. Composition according to either one of Claims 1 and 2, **characterized in that** the compound of the avermectin family is chosen from ivermectin, invermectin, avermectin, abamectin, doramectin, eprinomectin and selamectin.

4. Composition according to one of Claims 1 to 3, **characterized in that** the compound of the avermectin family is ivermectin.

5. Composition according to one of Claims 1 to 4, **characterized in that** the oily internal phase of the nanocapsules comprises a fatty substance which is liquid or semi-liquid at ambient temperature.

6. Composition according to one of Claims 1 to 5, **characterized in that** the oily internal phase of the nanocapsules comprises at least one fatty substance chosen from diisopropyl adipate, PPG 15 stearyl ether, octyldodecanol, C₁₂-C₁₅ alkyl benzoate, dicaprylyl ether, octyl palmitate, ethoxydiglycol, lanolin, benzyl benzoate, and mixtures thereof.

7. Composition according to Claim 5 or 6, **characterized in that** the fatty substance is present in an amount of between 90% and 99.99% by weight relative to the total weight of the internal phase.

8. Composition according to one of Claims 1 to 7, **characterized in that** the lipophilic surfactant which is solid at ambient temperature is present in an amount of between 0.1% and 10% by weight relative to the total weight of the composition, preferably between 1% and 5%.

9. Composition according to one of Claims 1 to 8, **characterized in that** it comprises a gelling agent.

10. Composition according to Claim 9, **characterized in that** the gelling agent is a cellulose-based derivative chosen from methylcellulose, ethylcellulose, hydroxypropylmethylcellulose, hydroxyethylcellulose, carboxymethylcellulose, hydroxymethylcellulose and hydroxypropylcellulose, taken alone or as a mixture.

11. Composition according to one of Claims 1 to 10, **characterized in that** it comprises in water, by weight relative to the total weight of the composition:
a) 0.1% to 5% of lipophilic surfactant which is solid at ambient temperature;
b) 1% to 20% of fatty substance which is liquid or semi-liquid at ambient temperature;
c) 0.1% to 5% of at least one avermectin;
d) 0 to 2% of gelling agent.

12. Composition according to Claim 11, **characterized in that** it comprises in water, by weight relative to the total weight of the composition:
a) 0.1% to 5% of lecithin;
b) 1% to 20% of diisopropyl adipate;
c) 0.1% to 5% of ivermectin;
d) 0 to 2% of cellulose-based derivative.

13. Composition according to one of Claims 1 to 12, **characterized in that** it is in a form suitable for topical administration.

14. Composition according to one of Claims 1 to 13, as a medicament.

15. Use of a composition according to one of Claims 1 to 13, for preparing a medicament intended for the treatment of dermatological conditions chosen from rosacea, common acne, seborrheic dermatitis, perioral dermatitis, acneiform eruptions, transient acantholytic dermatosis, and acne necrotica miliaris.

16. Use according to Claim 15, **characterized in that** the dermatological condition is rosacea.

17. Method for preparing a composition according to one of Claims 1 to 13, **characterized in that** it comprises the following steps:
(i) solubilization of the avermectin in a fatty substance which is liquid or semi-liquid at ambient temperature, in order to obtain the oily phase;
(ii) mixing of the hydrophilic compounds, in order to obtain the hydrophilic phase;
(iii) dispersion of the lipophilic surfactant in the oily phase obtained in (i) or in the hydrophilic phase obtained in (ii);
(iv) mixing of the oily phase with the hydrophilic phase;
(v) introduction of the mixture obtained in (iv) into a high-pressure homogenizer, in order to obtain a nanoemulsion.

18. Method according to Claim 17, **characterized in that** it comprises a step (vi) of addition of a gelling agent to the nanoemulsion obtained in (v).
